# EUROPEAN PATENT SPECIFICATION

(11) **EP 3 032 240 B1**
(45) Date of publication and mention of the grant of the patent: **22.01.2020**
(21) Application number: 15198732.8
(22) Date of filing: 09.12.2015
(51) Int. Cl.: G01N 17/04, G01N 33/28, G01N 17/00

(54) **SYSTEM AND METHOD FOR TESTING CORROSIVE EFFECT OF A HYDROCARBON ON A COMPONENT**
SYSTEM UND VERFAHREN ZUM TESTEN DES KORRODIERENDEN EFFEKTS EINES KOHLENWASSERSTOFFS AUF EINE KOMPONENTE
SYSTÈME ET PROCÉDÉ POUR TESTER LA CORROSIVITÉ D'UN HYDROCARBURE SUR UN COMPOSANT

(30) Priority: 11.12.2014 US 201462090597 P
(43) Date of publication of application: 15.06.2016
(73) Proprietor: Shell Internationale Research Maatschappij B.V., 2596 HR The Hague (NL)
(72) Inventor: BENNIS, Hanane Belmokaddem, Richmond, TX 77407 (US); SHEA, Timothy Michael, The Woodlands, TX 77380 (US); BAKER, Michael Lane, Richmond, TX 77407 (US)
(74) Representative: Shell Legal Services IP

(56) References cited:
- CA-A1- 2 227 382
- "ASTM D849-11 Standard Test Method for Copper Strip Corrosion by Industrial Aromatic Hydrocarbons", ASTM STANDARDS, 2011, XP008179704, DOI: 10.1520/D0849-11
- M L Meyer ET AL: "A Materials Compatibility and Thermal Stability Analysis of Common Hydrocarbon Fuels", , 1 January 2005 (2005-01-01), XP055262944, Retrieved from the Internet: URL:http://ntrs.nasa.gov/archive/nasa/casi .ntrs.nasa.gov/20050158752.pdf [retrieved on 2016-04-05]
- "Handbook of aviation fuel properties", , 1 January 1983 (1983-01-01), XP055263067, Retrieved from the Internet: URL:http://www.dtic.mil/dtic/tr/fulltext/u 2/a132106.pdf [retrieved on 2016-04-05]

## Description

### Field of the Invention

The present disclosure relates generally to hydrocarbons. More specifically, the present disclosure relates to techniques for testing hydrocarbons, such as fuels, additives, and/or lubricants.

### Background of the Invention

Hydrocarbons, such as fuel, may be used in machinery, such as engines, to provide power. For example, hydrocarbons may be housed in a fuel tank and passed via fuel lines to engines, and ignited to generate power. The composition of the fuel may affect the function of the equipment used with the fuel. In some cases, the hydrocarbons may damage equipment, such as the fuel tanks and fuel lines that contact the hydrocarbons during operation.

Tests have been developed for evaluating fuels. Examples of tests involving fuels are provided in US6125690 and JP2009264394. Standards have also been provided for evaluating fuels. For example, the American Society for Testing and Materials (ASTM) International has developed ASTM Designation: D7826-13 (available at www.astm.org) for evaluating gasoline.

"ASTM D849-11 Standard Test Method for Copper Strip Corrosion by Industrial Aromatic Hydrocarbons", ASTM Standards, 2011, XP008179704, DOI: 10.1520/D0849-11 discloses a tester for testing exposure of a copper strip to a hydrocarbon.

M.L.Meyer et al: "A Materials Compatibility and Thermal Stability Analysis of Common Hydrocarbon Fuels", 1 January 2005 (2005-01-01), XP055262944 discloses a compatibility tester for hydrocarbon fuels and various components.

CA 2 227 382 A1 discloses an automatic tester and an automated test method to determine the corrosivity of hydrocarbons when brought into contact with a copper strip.

### Summary of the Invention

In at least one aspect of the present invention there is provided a tester (108) for testing exposure of a hydrocarbon (102) on a component (112), the hydrocarbon stored in a fluid source (104), the tester comprising:
a test module (110) supporting the component thereabout, the test module operatively connectable to the fluid source to receive the hydrocarbon therefrom; and
a recirculation circuit (114) operatively connectable about the test module to recirculate the hydrocarbon thereabout whereby the hydrocarbon is passed over at least a portion of the component; and
at least one sensor (122) operatively configured to be connected to the fluid source, the test module, and the recirculation circuit; .
wherein the test module comprises a partial exposure housing (226b) having a component cavity shaped to fittingly receive an outer surface of the component, and
wherein the component is at least one of a coupon, a gasket, a tube, and combinations thereof.

In another aspect of the present invention there is provided a system for testing exposure of a hydrocarbon on a component. The system includes at least one fluid source having the hydrocarbon therein, a fluid circuit in fluid communication with the fluid source, and a plurality of testers operatively connectable to the fluid circuit to receive the hydrocarbon therefrom. Each of the testers includes a test module supporting the component thereabout and a plurality of recirculation circuits. Each of the recirculation circuits is operatively connectable to the fluid circuit about a corresponding one of the plurality of test modules to recirculate the hydrocarbon thereabout whereby the hydrocarbon is passed over at least a portion of the component.

### Brief Description of the Drawings

So that the above recited features and advantages of the disclosure may be understood in detail, a more particular description of the disclosure, briefly summarized above, may be had by reference to the embodiments thereof that are illustrated in the appended drawings. It is to be noted, however, that the appended drawings illustrate only typical embodiments of this disclosure and are, therefore, not to be considered limiting of its scope. The figures are not necessarily to scale, and certain features and certain views of the figures may be shown exaggerated in scale or in schematic in the interest of clarity and conciseness.
Figure 1 is a schematic diagram depicting a system with testers having test modules for testing exposure of a hydrocarbon on a component, in accordance with the present disclosure;
Figures 2A-2B are schematic diagrams depicting an immersion test module usable in the system of Figure 1, in accordance with the present disclosure;
Figures 3A-3B are schematic diagrams depicting a partial-exposure test module usable in the system of Figure 1, in accordance with the present disclosure;
Figure 4 is a schematic diagram depicting a flow-through test module usable in the system of Figure 1, in accordance with the present disclosure; and
Figures 5A and 5B are flow charts depicting methods for testing exposure of a hydrocarbon on a component, in accordance with the present disclosure.

### Detailed Description of the Invention

The description that follows includes exemplary apparatuses, methods, techniques, and instruction sequences that embody techniques of the inventive subject matter. However, it is understood that the described embodiments may be practiced without these specific details.

The disclosure relates to techniques for testing exposure of hydrocarbons, such as fuels and additives, on components selected from coupons, gaskets, tubes, and combinations thereof. The tests may be performed using a modular test system with one or more testers. Each tester includes a test module to support the component, and a recirculation circuit to recirculate the hydrocarbon about the test module. The hydrocarbon is passed via a fluid circuit about one or more testers. Sensors and/or visual detectors are provided to sense changes in fluid parameters (e.g., viscosity, temperature, flow rate, etc.) of the hydrocarbon, material parameters (e.g., color, material properties, etc.) of the components, and/or other portions of the system. Material tests (e.g., hardness, tensile, etc.) may also be performed on the components.

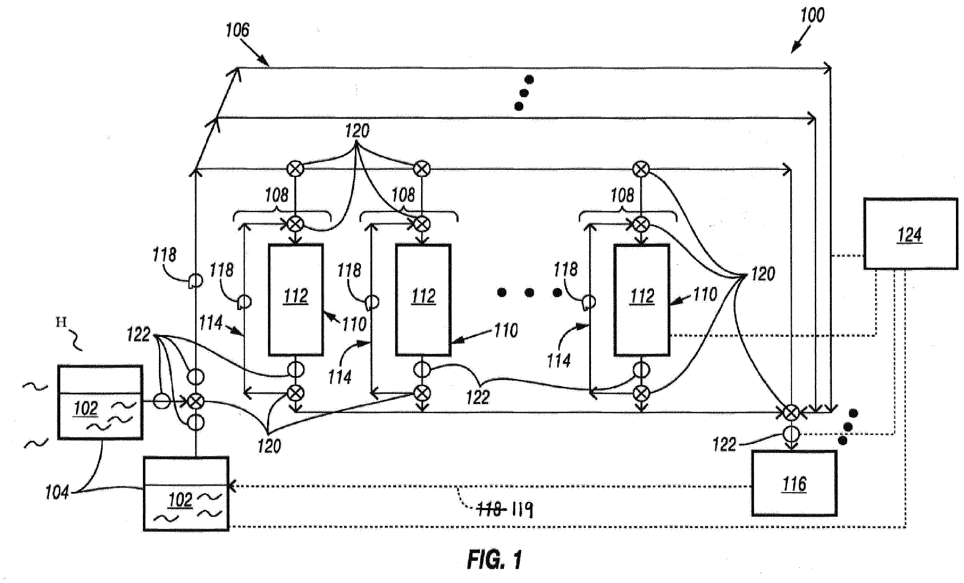

Figure 1 illustrates a test system 100 usable for evaluating a hydrocarbon 102. The test system 100 includes fluid sources 104, a fluid circuit 106, and testers 108. One or more fluid sources 104 are provided to store the hydrocarbon 102. The fluid sources 104 may have the same or different hydrocarbons.

The test system 100 is described with respect to exposure of a hydrocarbon to a component. The hydrocarbon may be, for example, a fuel, such as gasoline, diesel, and/or additives usable therewith. Other fuels may be envisioned, such as ethanol, biodiesel, propane, compressed natural gas, battery, hydrogen or fluid. An example hydrocarbon usable with the test system would be a fuel having a viscosity of from about 0.5 mm ²/sec to about 1,500 mm²/s or more, and a temperature of about 40 degrees C.

The fluid sources 104 are fluidly coupled to the fluid circuit 106. The fluid circuit 106 includes flowlines fluidly coupling the fluid source(s) 104 to one or more of the testers 108. The flowlines may be fluid lines made of materials, such as tygon tubing, stainless steel, etc. As also shown by H in Figure 1, the fluid sources 104 (and/or other portions of the fluid circuit 106) may be heated. Heat may be applied to the hydrocarbon 102 before or after passing through the fluid circuit 106.

The testers 108 include test modules 110 for supporting the components 112 for testing. The test modules 110 are positioned in fluid communication with the fluid circuit 106 to receive the hydrocarbon 102 therefrom and to expose at least a portion of the component 112 thereto as schematically shown.

A recirculation circuit 114 is provided about each of the test modules 110 to recirculate the hydrocarbon 102 therethrough as indicated by the arrows. The recirculation circuit 114 may be coupled to the test module 110 and/or the fluid circuit 106 to repeatedly pass the hydrocarbon 102 through the test module 110. Once complete the hydrocarbon 102 may be discharged from the tester 108.

A variety of flowlines of the fluid circuit 106 may be configured to accommodate a variety of configurations of the system 100. The flowlines as shown couples the fluid sources 104 to multiple test modules 108 that may be tested simultaneously, in pattern (e.g., series), or independently. On or more sets, rows, columns, or other patterns of test modules 108 may be provided. As indicated by the ellipses, the system 100 may be expandable to accommodate any combination of components of the system as provided herein.

The system 100 may be modular to provide removable components 112 and/or reconfigurable to accommodate a variety of arrangements. One or more test modules 110 may be provided about the system 100 to permit testing of one or more components and/or hyrdocarbons at the same time for comparison. In some cases, a test cycle of various components and/or hydrocarbons may be varied to provide desired experiments designed to determine various characteristics.

The flowlines of the fluid circuit 106 may also be configured to provide for discharge of the fluid from the testers 108 to a waste bin 116. As indicated by dashed line 119, a recirculation flowline may be provided to pass hydrocarbon 102 from waste bin 116 back to the fluid source 104 and/or the fluid circuit 106 for reuse. The hydrocarbon 102 may be passed to one or more waste bins 116 and/or recirculated to one of more of the testers 108 as desired.

The fluid circuit 106 may be provided with a variety of flow control devices, such as pumps 118, valves 120, etc. Pump 118 in fluid circuit 106 may be used to pump the hydrocarbon 102 through the flow circuit 106. Pump 118 in recirculation circuit 114 may be used to pump the hydrocarbon 102 through the test module 108. Valves 120 may be positioned at various locations along the fluid circuit 106 to direct hydrocarbon 102 to desired locations. For example, on completion of a test, one or more testers 108 may be fluidly isolated by valves and/or be removable from the fluid circuit 106.

The fluid circuit 106 provides a closed system usable with a variety of fluid, including flammable, toxic, evaporable, or other sensitive fluids. For example, the fluid may be an aviation gasoline prone to evaporation which may affect the results (e.g., where evaporation of the aviation gas may cause the percent of heavier components in the fluid blend higher).

The system 100 is also be provided with sensors 122. The sensors 122 may be, for example, gauges, monitors, electrodes, or other sensors capable of measuring a variety of system (e.g., flow rate), fluid (e.g., temperature, viscosity, composition, etc.) and/or material (e.g., material, temperature) parameters. The sensors 122 may be positioned, for example, along the flowlines of the fluid circuit 106 and/or the recirculation circuit 114. The sensors 112 may be coupled to a central unit 124 for collection and/or analysis.

The central unit 124 may be provided with a variety of communication, power, storage, control, and/or analysis functions. For example, the central unit 124 may include a processor (e.g., central processing unit (CPU)), database, transceiver, controller, and/or other devices. The central unit 124 may be located at, or a distance from, the fluid circuit 106 and in communication therewith. One or more central units 124 at various locations may be provided. The central units 124 may be used, for example, to collect, compare, and report on various data collected involving one or more testers, hydrocarbons, and/or components.

The test system 100 may be operated automatically using central unit 124 and/or manually. The hydrocarbon 102 is passed through the fluid circuit 106 to the test modules 110. The recirculation loop 114 is used to recirculate the same hydrocarbon through the test module(s) 110 one or more times. The hydrocarbon 102 may continue to pass through the test module 110 one or more times until a test is completed.

The test may be completed based on a predetermined test time or upon detection of a change in the system 100. For example, the sensors 122 may detect a change in material and/or fluid parameters indicating the test should be terminated. In some cases, visual inspection of the component 112 may indicate that a color change has occurred. Once completed, the test may be automatically or manually terminated.

Once terminated, the test module 110 may be fluidly isolated from the fluid circuit 106 by valves 120 and the hydrocarbon 102 may be allowed to drain from the test module to waste bin 116. The component 112 may be visible through the test module 110, or removed from the test module 110 for inspection and/or further testing. Further testing may include, for example, material testing, such as hardness, tensile, compressive, or other known material tests. Data from these tests may be provided to the central unit 124 for further analysis alone or with other data, such as data collected during the test by system 100. The data may be stored, distributed, analyzed, reported, etc.

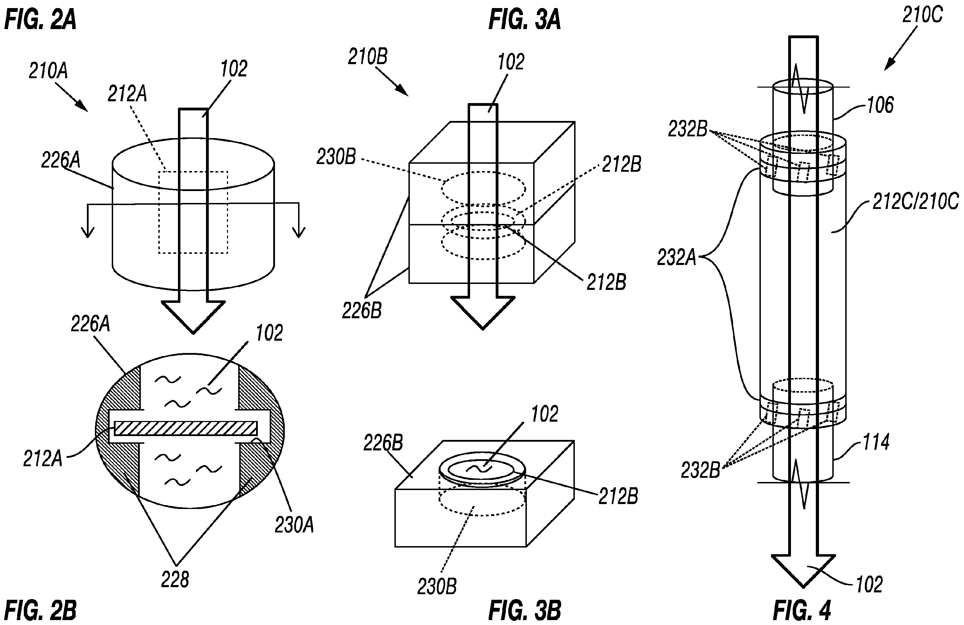

Figures 2A - 4 show various example test modules 210a-c that may be used with various components 212a-c. Figures 2A - 2B depict an example immersion test module 210a. Figures 3A - 3C depict an example partial-exposure test module 210b. Figure 4 depicts an example flow-through test module 210c. Each example test module 210a-c supports the component 212a-c, respectively, therein such that the hydrocarbon 102 passes along a desired portion of the component.

In the example of Figures 2A - 2B, the component 212a is a coupon (e.g., metal plate) immersed within the test module 210a. The test module 210a includes a housing 226a with supports 228 therein. The housing 226a as shown is elliptical in shape, but could be in any form. The supports 228 are depicted as defining an immersion receptacle 230a for receiving the coupon 212a. As shown, the housing 226a loosely receives the coupon 212a such that most if not all portions of the coupon 212a are exposable to (e.g., immersed in) the hydrocarbon 102 passing through the housing 226a as indicated by the arrow. In this configuration, the coupon 212a may be immersed within the hydrocarbon 102.

In the example of Figures 3A - 3B, the component 212b is an elliptical gasket insertable into the test module 210b. The test module 210b includes a two-portion housing 226b. As shown, the housing 226b is rectangular with two connectable portions, with each portion having an interference fit receptacle 230b shaped to snugly receive an outer surface of the gasket 212b. The housing portions are matable to fittingly receive the gasket 212b therein such that fluid passing through the housing 226b passes along an inner surface of the gasket 212b, but cannot engage an outer surface of the gasket 212b. Thus, the outer surface of the gasket 212b is in contact with a surface of the housing 226b along the receptacle 230b to prevent hydrocarbon contact therealong, and only the inner surface of the gasket 212b may be exposed.

While Figures 3A and 3B show a rectangular housing 226b in two portions, the housing can be any shape with one or more housing portions to receive and/or support the gasket 212b therein. The receptacle 230b may be shaped to receive the component for partial or full exposure to the hydrocarbon 102. The receptacle 230b may be larger than the component or a smaller component may be placed in a larger receptacle 230b to permit full immersion when desired.

In the example of Figure 4, the component 212c is a tube connectable to a flowline of the flow circuit 106 and the recirculation circuit 114. In this case, the tube 212c is its own self-contained module 210c. The tube 212c is connected by connectors 232a, b to the flowlines of the flow circuits 106 and/or recirculation loop 114. In this configuration, the tube 212c is connected so that hydrocarbon 102 may pass through the flow circuit 106, through tube 212c, and out the recirculation loop 114 such that an inner surface of the tube 212c is exposed to the hydrocarbon 102.

As shown in Figure 4, the connectors 232a are clamps forming a band tightened around the tube 212c to secure the tube 212c to the flowlines. As also shown, the connectors 232b are barbs 232b also connecting the tube 212c to the flowlines. The barbs 232b may be on the tube 212c and/or the flowlines. The barbs 232b may have points to grip the tube 212c and secure the tube 212c to the flowline. One or more of a variety of connectors, such as clamps and/or barbs, may be used to connect the components/test modules 212c/210a about the flow circuit 106 and/or recirculation circuit 114.

While Figures 2A-4 show example test modules that may be used in the system 100 and/or with various components, it will be appreciated that a variety of test modules may be used to support one or more components for exposure to hydrocarbon. The test modules may be configured to permit exposure of part, or all, of the component to the hydrocarbon.

The components are components that are exposed to the hydrocarbon, and are selected from coupons of material used for fuel tanks, seals (or gaskets), tubes, and combinations thereof. The test modules may be made of any material, such as glass, stainless steel, aluminum, etc.) For example, the test modules may optionally be clear or have a window to permit visual inspection of the components during testing. Visual inspection may be used to monitor changes and may indicate completion of a test. Various combinations of the test modules may also be provided.

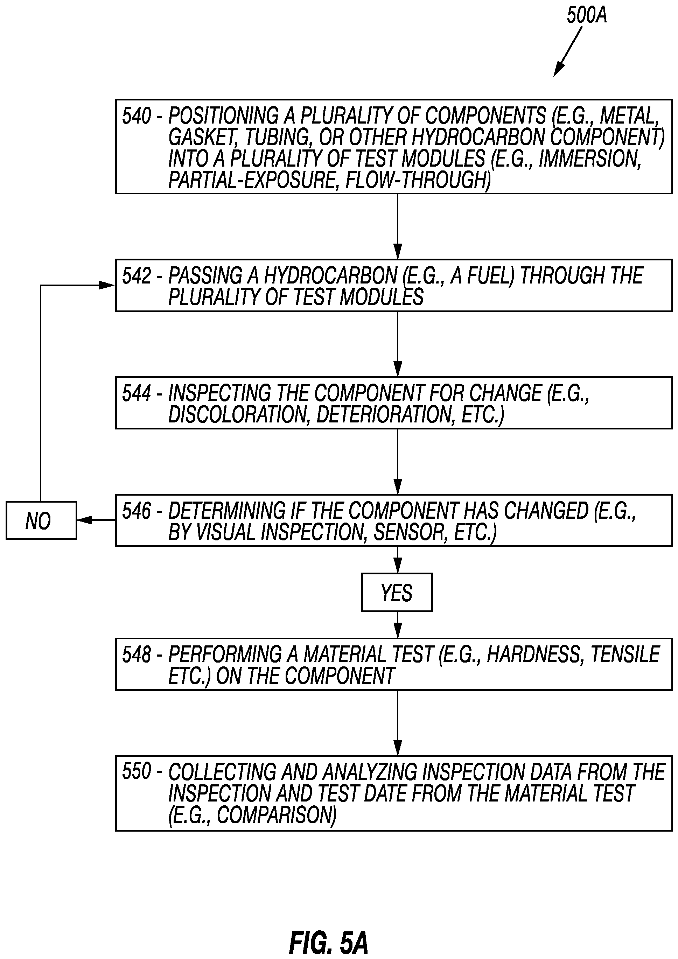

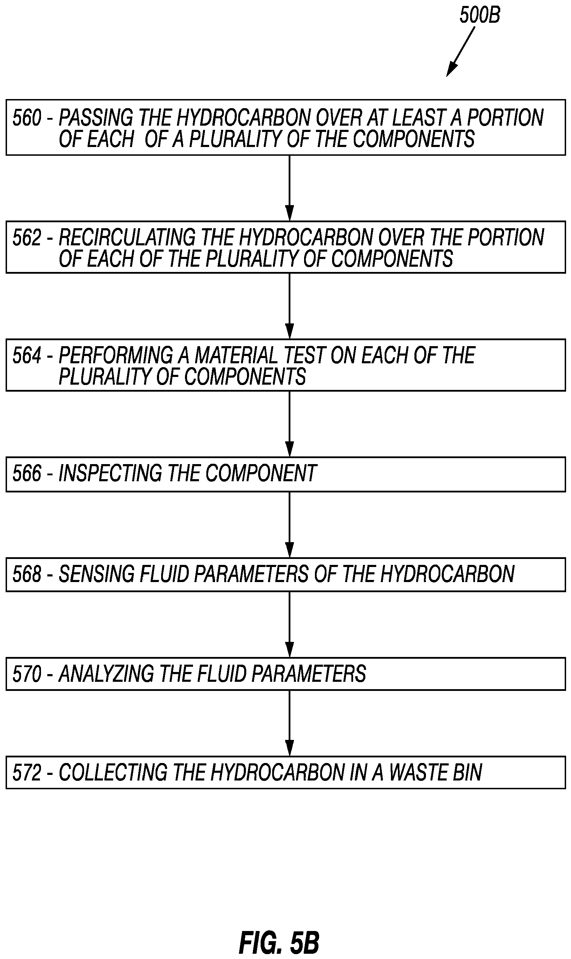

Figures 5A and 5B depict methods 500a,b outside of the scope of the present invention of testing exposure of a hydrocarbon on a component, such as the hydrocarbon and components shown herein. Flowchart 500a may involve 540 - positioning a plurality of components (e.g., metal, gasket, tubing, or other hydrocarbon component) into a plurality of test modules (e.g., immersion, partial-exposure, and flow-through). Further, may also involve 542 - passing a hydrocarbon (e.g., a fuel) through the plurality of test modules, 544 - inspecting (e.g., visually inspecting, removing from the component for inspection, inspecting with sensor) the component for change (e.g., discoloration, deterioration, etc.), and 546 - determining if the component has changed (e.g., by visual inspection, sensor, etc.) The passing 542 may be performed one or more times. In some cases 544-546 may be skipped and the passing 542 may be performed for a given time frame before proceeding to 548-550.

If the component has not changed, 542-546 may continue until a change is detected. If the component has changed, the procedure may continue with 548 - performing a material test (e.g., hardness, tensile, etc.) on the component, and 550 - collecting and analyzing inspection data from the inspection and test date from the material test (e.g., comparison).

Flowchart 500b involves 560 - passing the hydrocarbon over at least a portion of each of a plurality of the components, 562 - recirculating the hydrocarbon over the portion of each of the plurality of components, and 564 - performing a material test on each of the plurality of components.

The procedure may also involve 566 - inspecting the component (e.g., by visual or sensors), 568 - sensing fluid parameters of the hydrocarbon (e.g., by sensors 122), 570 - analyzing the fluid parameters (e.g., by central unit 124), and/or 572 - collecting the hydrocarbon in a waste bin (e.g., 116). The analyzing may involve comparing each of the plurality of components. The recirculating may involve recirculating the hydrocarbon about each one of the plurality of components (e.g., by 114) and/or recirculating the hydrocarbon about all of the plurality of components (e.g., by 118).

The steps may be repeated as desired and performed in any order.

While the embodiments are described with reference to various implementations and exploitations, it will be understood that these embodiments are illustrative and that the scope of the inventive subject matter is not limited to them. Many variations, modifications, additions and improvements are possible. For example, one or more testers and/or test modules with one or more test components and/or hydrocarbons tested by visual, sensor, and/or material testing over time as described herein. The person skilled in the art will readily understand that, while the invention is illustrated making reference to one or more a specific combinations of features and measures, many of those features and measures are functionally independent from other features and measures such that they can be equally or similarly applied independently in other embodiments or combinations.

Plural instances may be provided for components, operations or structures described herein as a single instance. In general, structures and functionality presented as separate components in the exemplary configurations may be implemented as a combined structure or component. Similarly, structures and functionality presented as a single component may be implemented as separate components.

## Claims

1. A tester (108) for testing exposure of a hydrocarbon (102) on a component (112), the hydrocarbon stored in a fluid source (104), the tester comprising:
a test module (110) supporting the component thereabout, the test module operatively configured to be connected to the fluid source to receive the hydrocarbon therefrom; and
a recirculation circuit (114) operatively configured to be connected to the test module to recirculate the hydrocarbon thereabout whereby the hydrocarbon is passed over at least a portion of the component; and
at least one sensor (122) operatively configured to be connected to the fluid source, the test module, and the recirculation circuit; .
wherein the test module comprises a partial exposure housing (226b) having a component cavity shaped to fittingly receive an outer surface of the component, and wherein the component is at least one of a coupon, a gasket, a tube, and combinations thereof.

2. A tester according to claim 1, wherein the test module comprises an immersion housing (226a) having a component support to supportingly receive the component.

3. A tester according to claim 1 or 2, wherein the test module comprises flow-through connectors (232) operatively configured to connect the component to the fluid source and the recirculation circuit, preferably the flow-through connector comprises at least one of a clamp and a barb.

4. A tester according to any of claims 1 to 3, wherein the recirculation circuit comprises a flowline operatively configured to be connected to an upstream end and a downstream end of the test module to recirculate the hydrocarbon thereabout.

5. A tester according to any of claims 1 to 4, wherein the hydrocarbon is a fuel having a viscosity of at least 0.5 mm ²/sec at 40 degrees C.

6. A tester according to any of claims 1 to 5, wherein the test module is at least partially clear to enable visual inspection of the component and wherein the test module comprises at least one of glass, stainless steel, and aluminum.

7. A system (100) for testing exposure of a hydrocarbon (102) on a component (112), the system comprising:
at least one fluid source (104) having the hydrocarbon therein;
a fluid circuit (106) in fluid communication with the fluid source; and
a plurality of testers according to claim 1 operatively configured to be connected to the fluid circuit to receive the hydrocarbon therefrom.

8. A system according to claim 7, further comprising at least one waste unit (116) operatively configured to be connected to the fluid circuit.

9. A system according to claims 7 or 8, further comprising at least one of a pump (118), a valve (120), and a flowline.

10. A system according to any of claims 7 to 9, further comprising an analyzer operatively configured to be connected to at least one of the at least one fluid source, the fluid circuit, the test modules, and the recirculation circuits.

## Patentansprüche

1. Tester (108) zum Testen der Exposition eines Kohlenwasserstoffs (102) an einer Komponente (112), wobei der Kohlenwasserstoff in einer Fluidquelle (104) gespeichert ist, wobei der Tester umfasst:
ein Testmodul (110), das die Komponente darüber trägt, wobei das Testmodul betriebsfähig ausgelegt ist, um mit der Fluidquelle verbunden zu werden, um den Kohlenwasserstoff daraus aufzunehmen; und
einen Umwälzkreislauf (114), der betriebsfähig ausgelegt ist, um mit dem Testmodul verbunden zu werden, um den Kohlenwasserstoff um dieses herum umzuwälzen, wodurch der Kohlenwasserstoff über mindestens einen Teil der Komponente geleitet wird; und
mindestens einen Sensor (122), der betriebsfähig ausgelegt ist, um mit der Fluidquelle, dem Testmodul und dem Umwälzkreislauf verbunden zu werden;
wobei das Testmodul ein Gehäuse zur teilweisen Exposition (226b) mit einem Komponentenhohlraum umfasst, der so geformt ist, dass er eine Außenfläche der Komponente passend aufnimmt, und wobei die Komponente entweder ein Abschnitt, eine Dichtung, ein Rohr und/oder Kombinationen davon ist.

2. Tester nach Anspruch 1, wobei das Testmodul ein Tauchgehäuse (226a) mit einem Komponententräger zum stützenden Aufnehmen der Komponente aufweist.

3. Tester nach Anspruch 1 oder 2, wobei das Testmodul Durchflussverbinder (232) umfasst, die betriebsfähig ausgelegt sind, um die Komponente mit der Fluidquelle und dem Umwälzkreislauf zu verbinden, wobei der Durchflussverbinder vorzugsweise mindestens entweder eine Klemme oder einen Widerhaken aufweist.

4. Tester nach einem der Ansprüche 1 bis 3, wobei der Umwälzkreislauf eine Strömungsleitung umfasst, die betriebsfähig so ausgelegt ist, dass sie mit einem stromaufwärtigen Ende und einem stromabwärtigen Ende des Testmoduls verbunden ist, um den Kohlenwasserstoff um dieses herum umzuwälzen.

5. Tester nach einem der Ansprüche 1 bis 4, wobei der Kohlenwasserstoff ein Kraftstoff mit einer Viskosität von mindestens 0,5 mm²/s bei 40 °C ist.

6. Tester nach einem der Ansprüche 1 bis 5, wobei das Testmodul mindestens teilweise durchsichtig ist, um eine Sichtprüfung der Komponente zu ermöglichen, und wobei das Testmodul mindestens eines aus Glas, Edelstahl und Aluminium umfasst.

7. System (100) zum Testen der Exposition eines Kohlenwasserstoffs (102) an einer Komponente (112), wobei das System umfasst:
mindestens eine Fluidquelle (104) mit dem Kohlenwasserstoff darin;
einen Fluidkreislauf (106) in Fluidverbindung mit der Fluidquelle; und
mehrere Tester nach Anspruch 1, die betriebsfähig so konfiguriert sind, dass sie mit dem Fluidkreislauf verbunden sind, um den Kohlenwasserstoff daraus aufzunehmen.

8. System nach Anspruch 7, ferner umfassend mindestens eine Abfalleinheit (116), die betriebsfähig ausgelegt ist, um mit dem Fluidkreislauf verbunden zu werden.

9. System nach Anspruch 7 oder 8, das ferner mindestens entweder eine Pumpe (118), ein Ventil (120) und/oder eine Strömungsleitung umfasst.

10. System nach einem der Ansprüche 7 bis 9, das ferner einen Analysator umfasst, der betriebsfähig ausgelegt ist, um mit mindestens entweder der mindestens einen Fluidquelle, dem Fluidkreis, den Testmodulen oder den Umwälzkreisläufen verbunden zu werden.

## Revendications

1. Testeur (108) servant à tester l'exposition d'un hydrocarbure (102) sur un composant (112), l'hydrocarbure étant stocké dans une source de fluide (104), le testeur comprenant :
un module de test (110) supportant le composant à proximité de celui-ci, le module de test étant fonctionnellement conçu pour être relié à la source de fluide afin de recevoir l'hydrocarbure de celle-ci ; et
un circuit de recirculation (114) fonctionnellement conçu pour être relié au module de test afin de faire recirculer l'hydrocarbure autour de celui-ci, l'hydrocarbure étant passé sur au moins une partie du composant ; et
au moins un capteur (122) fonctionnellement conçu pour être relié à la source de fluide, au module de test et au circuit de recirculation ;
dans lequel le module de test comprend un boîtier d'exposition partielle (226b) comportant une cavité de composant formée pour recevoir de manière ajustée une surface extérieure du composant, et dans lequel le composant est un coupon et/ou un joint et/ou un tube et/ou des combinaisons de ceux-ci.

2. Testeur selon la revendication 1, dans lequel le module de test comprend un boîtier d'immersion (226a) comportant un support de composant destiné à recevoir le composant de manière supportée.

3. Testeur selon la revendication 1 ou 2, dans lequel le module de test comprend des connecteurs à écoulement traversant (232) fonctionnellement conçus pour relier le composant à la source de fluide et au circuit de recirculation, de préférence le connecteur à écoulement traversant comprend une pince et/ou une barbe.

4. Testeur selon l'une quelconque des revendications 1 à 3, dans lequel le circuit de recirculation comprend une ligne d'écoulement fonctionnellement conçue pour être reliée à une extrémité amont et à une extrémité aval du module de test afin de faire recirculer l'hydrocarbure autour de celui-ci.

5. Testeur selon l'une quelconque des revendications 1 à 4, dans lequel l'hydrocarbure est un carburant présentant une viscosité d'au moins 0,5 mm²/s à 40 °C.

6. Testeur selon l'une quelconque des revendications 1 à 5, dans lequel le module de test est au moins partiellement transparent pour permettre l'inspection visuelle du composant, et dans lequel le module de test comprend le verre et/ou l'acier inoxydable et/ou l'aluminium.

7. Système (100) de test de l'exposition d'un hydrocarbure (102) sur un composant (112), le système comprenant :
au moins une source de fluide (104) contenant l'hydrocarbure ;
un circuit de fluide (106) en communication fluidique avec la source de fluide ; et
une pluralité de testeurs selon la revendication 1, fonctionnellement conçus pour être reliés au circuit de fluide afin de recevoir l'hydrocarbure de celui-ci.

8. Système selon la revendication 7, comprenant en outre au moins une unité de gestion de déchets (116) fonctionnellement conçue pour être reliée au circuit de fluide.

9. Système selon les revendications 7 ou 8, comprenant en outre une pompe (118) et/ou une vanne (120) et/ou une ligne d'écoulement.

10. Système selon l'une quelconque des revendications 7 à 9, comprenant en outre un analyseur fonctionnellement conçu pour être relié à une source de fluide et/ou au circuit de fluide et/ou aux modules de test et/ou aux circuits de recirculation.
